# EUROPEAN PATENT APPLICATION

(11) **EP 2 907 507 A1**
(43) Date of publication of application: **19.08.2015**
(21) Application number: 14155466.7
(22) Date of filing: 17.02.2014
(51) Int. Cl.: A61K 9/20, A61K 31/4545

(54) **Pharmaceutical composition comprising apixaban**

(71) Applicant: SANDOZ AG, 4056 Basel (CH)
(72) Inventor: The designation of the inventor has not yet been filed
(74) Representative: Kluschanzoff, Harald

(57) **Abstract**

The present invention relates to a pharmaceutical composition comprising apixaban, in particular to a pharmaceutical composition comprising apixaban and a polymer having low viscosity as binder, and to a process for its preparation. The pharmaceutical composition is particularly useful as a medicament, especially for the treatment or prevention of a thromboembolic disorder.

## Description

### Field of invention

The present invention relates to a pharmaceutical composition comprising apixaban and a particularly selected polymer. The present invention also relates to a process for the preparation of pharmaceutical composition comprising apixaban and the particularly selected polymer. The pharmaceutical composition is particularly useful as a medicament, especially for the treatment or prevention of a thromboembolic disorder.

### Description of Background Art

Apixaban, 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxamide), shown in formula (1) is a factor Xa inhibitor used for the treatment or prevention of a thromboembolic disorder. Apixaban was disclosed in U.S. Patent No. 6,967,208.

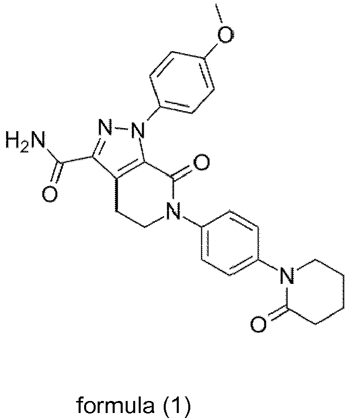

Apixaban is known to be poorly soluble. This results in slow and incomplete drug release and poor bioavailability. Therefore, there is a need to provide a pharmaceutical composition comprising apixaban that exhibits improved dissolution rate of drug substance.

In this regard, WO 2011/106478 discloses a composition comprising crystalline apixaban particles having a maximally limited mean particle size and a pharmaceutically acceptable diluent or carrier. To this end, the prior art proposed compositions require the particle size of active ingredient to be controlled to meet appropriate dissolution rate.

Therefore, there is an unmet need for new pharmaceutical composition comprising apixaban that exhibits improved dissolution rate, as a property optionally and preferably independent from a particle size that may be used.

### Summary of the invention

The aspects, advantageous features and preferred embodiments of the present invention summarized in the following items, respectively alone or in combination, further contribute to solving the object of the invention:
1. A pharmaceutical composition comprising apixaban and a polymer having low viscosity as binder.
2. The pharmaceutical composition of Item 1, wherein the polymer having low viscosity has a dynamic viscosity of 2% aqueous solution at 20°C up to about 25 mPa·s.
3. The pharmaceutical composition of Item 1 or 2, wherein for a given polymer type, a species having relatively lower viscosity is selected, measured as a dynamic viscosity of 2% aqueous solution at 20°C.
4. The pharmaceutical composition of any one of preceding Items, wherein the polymer having low viscosity is selected from the group consisting of povidone, hypromellose, hydroxypropyl cellulose, polyvinyl alcohol, copovidone, polyvinyl alcohol-polyethylene glycol graft copolymer, respectively having low viscosity, and a mixture thereof.
5. The pharmaceutical composition of any one of preceding Items, wherein the polymer having low viscosity has an average molecular weight of up to about 200000.
6. The pharmaceutical composition of any one of preceding Items, wherein the polymer having low viscosity is povidone with a dynamic viscosity of 10% aqueous solution at 25°C up to about 10 mPa·s.
7. The pharmaceutical composition of any one of preceding Items, wherein the polymer having low viscosity is povidone with K-value up to about 32.
8. The pharmaceutical composition of any one of preceding Items, wherein the polymer having low viscosity is povidone having an average molecular weight of up to about 55000.
9. The pharmaceutical composition of any one of preceding Items, wherein povidone is selected from the group consisting of K12, K17, K25, K30 and a mixture thereof.
10. The pharmaceutical composition of Item 9, wherein povidone is K25.
11. The pharmaceutical composition of any one of Items 1 to 5, wherein the polymer having low viscosity is hypromellose with a dynamic viscosity of 2% aqueous solution at 20°C up to about 20 mPa·s.
12. The pharmaceutical composition of any one of Items 1 to 5 and 11, wherein the polymer having low viscosity is hypromellose having an average molecular weight of up to about 100000.
13. The pharmaceutical composition of any one of Items 1 to 5 and 11 to 12, wherein hypromellose is selected from the group consisting of substitution types of hypromellose 2208, 2910, 2306, and a mixture thereof.
14. The pharmaceutical composition of Item 13, wherein hypromellose is selected from the group consisting of Pharmacoat 603, Pharmacoat 645, Pharmacoat 606, Pharmacoat 615, Pharmacoat 904, Methocel K3 Premium LV, Methocel E3 Premium LV, Methocel E5 Premium LV, Methocel E6 Premium LV , Methocel E15 Premium LV, Benecel E3, Benecel E6, Benecel E15, Benecel A15 LV and a mixture thereof.
15. The pharmaceutical composition of Item 14, wherein hypromellose is Pharmacoat 603.
16. The pharmaceutical composition of any one of Items 1 to 5, wherein the polymer having low viscosity is hydroxypropyl cellulose with a dynamic viscosity of 2% aqueous solution at 20°C up to about 25 mPa·s.
17. The pharmaceutical composition of any one of Items 1 to 5, wherein the polymer having low viscosity is hydroxypropyl cellulose with a dynamic viscosity of 5% aqueous solution at 25°C up to about 150 mPa·s.
18. The pharmaceutical composition of any one of Items 1 to 5, wherein the polymer having low viscosity is hydroxypropyl cellulose with a dynamic viscosity of 10% aqueous solution at 25°C up to about 700 mPa·s.
19. The pharmaceutical composition of any one of Items 1 to 5 and 16 to 18, wherein the polymer having low viscosity is hydroxypropyl cellulose having an average molecular weight of up to about 100000.
20. The pharmaceutical composition of any one of Items 1 to 5 and 16 to 19, wherein hydroxypropyl cellulose is selected from the group consisting of Klucel ELF, Klucel EF, Klucel EXF, Klucel LF, Nisso HPC-LM, Nisso HPC-L, Nisso HPC-SL, Nisso HPC-SSL and a mixture thereof.
21. The pharmaceutical composition of any one of Items 1 to 5, wherein the polymer having low viscosity is polyvinyl alcohol with a dynamic viscosity of 4% aqueous solution at 20°C up to about 40 mPa·s.
22. The pharmaceutical composition of any one of Items 1 to 5 and 21, wherein the polymer having low viscosity is polyvinyl alcohol having an average molecular weight of up to about 200000.
23. The pharmaceutical composition of any one of Items 1 to 5 and 21 to 22, wherein the polyvinyl alcohol is selected from the group consisting of PVA EMPROVE® 4-88, PVA EMPROVE® 5-88, PVA EMPROVE® 8-88, PVA EMPROVE® 28-99, PVA EMPROVE® 40-88, Mowiol® 3-96, Mowiol® 4-88, Mowiol® 4-98, Mowiol® 9-98, Mowiol® 18-88, Mowiol® 40-88, and a mixture thereof.
24. The pharmaceutical composition of any one of Items 1 to 5, wherein the polymer having low viscosity is copovidone (polyvinylpyrrolidone-vinyl acetate copolymer) with a dynamic viscosity of 10% aqueous solution at 25°C up to about 10 mPa·s.
25. The pharmaceutical composition of any one of Items 1 to 5 and 24, wherein the polymer having low viscosity is copovidone with K-value up to about 35.
26. The pharmaceutical composition of any one of Items 1 to 5 and 24 to 25, wherein the polymer having low viscosity is copovidone having an average molecular weight of up to about 80000.
27. The pharmaceutical composition of any one of Items 1 to 5 and 24 to 26, wherein copovidone is selected from the group consisting of Kollidon VA 64, Luviskol VA, Plasdone S-630 and a mixture thereof.
28. The pharmaceutical composition of any one of Items 1 to 5, wherein the polymer having low viscosity is polyvinyl alcohol-polyethylene glycol graft copolymer (Ethylene Glycol and Vinyl Alcohol Grafted Copolymer, Macrogol Poly(Vinyl Alcohol) Grafted Copolymer) with a dynamic viscosity of 20% aqueous solution at 25°C up to about 250 mPa·s.
29. The pharmaceutical composition of any one of Items 1 to 5, wherein the polymer having low viscosity is polyvinyl alcohol-polyethylene glycol graft copolymer (Ethylene Glycol and Vinyl Alcohol Grafted Copolymer, Macrogol Poly(Vinyl Alcohol) Grafted Copolymer) with a dynamic viscosity of 10% aqueous solution at 25°C up to about 20 mPa·s.
30. The pharmaceutical composition of any one of Items 1 to 5, wherein the polymer having low viscosity is polyvinyl alcohol-polyethylene glycol graft copolymer (Ethylene Glycol and Vinyl Alcohol Grafted Copolymer, Macrogol Poly(Vinyl Alcohol) Grafted Copolymer) with a dynamic viscosity of 5% aqueous solution at 25°C up to about 10 mPa·s.
31. The pharmaceutical composition of any one of Items 1 to 5 and 28 to 30, wherein the polymer having low viscosity is polyvinyl alcohol-polyethylene glycol graft copolymer having an average molecular weight of up to about 60000.
32. The pharmaceutical composition of any one of Items 1 to 5 and 28 to 31, wherein the polyvinyl alcohol-polyethylene glycol graft copolymer is Kollicoat IR.
33. The pharmaceutical composition of any one of preceding Items, further comprising a surfactant.
34. The pharmaceutical composition of Item 33, wherein the surfactant is selected from the group consisting of polyoxyethylene stearates, polyoxyethylene alkyl ethers, sorbitan fatty acid esters, poloxamers, polyoxyethylene castor oil derivatives, phospholipids, sodium lauryl sulphate, polysorbate (polyoxyethylene sorbitan fatty acid esters) and a mixture thereof.
35. The pharmaceutical composition of Item 34, wherein the surfactant is selected from the group consisting of sodium lauryl sulphate, polysorbate and a mixture thereof.
36. The pharmaceutical composition of any one of preceding Items, wherein the polymer having low viscosity is used as a wet granulation binder.
37. The pharmaceutical composition of any one of preceding Items, comprising apixaban having a particle size distribution of d_{0.9} 5 - 150 µm, preferably 30 - 130 µm, more preferably 40 - 120 µm.
38. The pharmaceutical composition of Item 37, comprising apixaban having a particle size distribution of d_{0.9} 50 - 100 µm.
39. The pharmaceutical composition of any one of preceding Items, further comprising pharmaceutically acceptable excipients selected from the group consisting of tableting fillers, disintegrants, glidants, lubricants, film coating agents and a mixture thereof.
40. The pharmaceutical composition of Item 39, wherein the tableting filler is selected from the group consisting of lactose, microcrystalline cellulose, starch, starch derivatives, sugars, sugar alcohols, inorganic fillers and a mixture thereof.
41. The pharmaceutical composition of Item 39, wherein the disintegrant is selected from the group consisting of crosscarmellose sodium, sodium starch glycolate, other starch derivatives, crospovidone, low substituted hydroxypropyl cellulose, ion exchange resins, magnesium aluminometasilicate and a mixture thereof.
42. The pharmaceutical composition of Item 39, wherein the glidant is selected from the group consisting of talc, silicon dioxide, magnesium silicate, sodium stearate and a mixture thereof.
43. The pharmaceutical composition of Item 39, wherein the lubricant is selected from the group consisting of magnesium stearate, sodium stearyl fumarate, glyceryl behenate, stearic acid, calcium stearate and a mixture thereof.
44. The pharmaceutical composition of Item 39, wherein the film coating agent is selected from the group consisting of polyvinyl alcohol, hypromellose, hydroxypropyl cellulose and a mixture thereof.
45. The pharmaceutical composition of any one of preceding Items, which exhibits dissolution properties that more than 80%, preferably more than 85%, more preferably more than 90% of apixaban is dissolved in 30 minutes in 900 ml of 0.1 M HCl solution.
46. A pharmaceutical composition as defined in any one of preceding Items for use in treating or preventing a thromboembolic disorder.
47. A process of manufacturing the pharmaceutical composition of any one of preceding Items, comprising mixing apixaban and a polymer having low viscosity.
48. The process of Item 47, which involves a wet granulation process using said polymer having low viscosity as a wet granulation binder.
49. The process of Item 47 or 48, comprising:
   applying water or a granulation dispersion onto a blend comprising apixaban and/or a polymer having low viscosity,
   wherein the granulation dispersion is prepared by dispersing apixaban and/or a polymer having low viscosity in a solvent.
50. The process of any one of Items 47 to 49, comprising further adding pharmaceutically acceptable excipients or a surfactant or both, to a granulation dispersion or to a blend comprising apixaban and/or a polymer having low viscosity.
51. The process of any one of Items 47 to 50, comprising:
   a. providing a granulation dispersion wherein apixaban and/or a polymer having low viscosity is dissolved or dispersed in water; and
   b. providing wet granulates by spraying or sprinkling said granulation dispersion obtained from step a onto a blend comprising apixaban and/or a polymer having low viscosity, and pharmaceutically acceptable excipients.
52. The process of any one of Items 47 to 50, comprising providing wet granulates by spraying or sprinkling water onto a blend comprising apixaban, a polymer having low viscosity and pharmaceutically acceptable excipients.
53. The process of any one of Items 47 to 50, comprising:
   a. providing a granulation dispersion wherein apixaban and/or a polymer having low viscosity and/or a surfactant is dissolved or dispersed in water; and
   b. providing wet granulates by spraying or sprinkling said granulation dispersion obtained from step a onto a blend comprising apixaban and/or a polymer having low viscosity and/or a surfactant, and pharmaceutically acceptable excipients.
54. The process of any one of Items 47 to 50, comprising providing wet granulates by spraying or sprinkling water onto a blend comprising apixaban, a polymer having low viscosity, a surfactant and pharmaceutically acceptable excipients.
55. The process of any one of Items 47 to 54, further comprising the steps of:
   a. drying wet granulates obtained after spraying or sprinkling;
   b. providing a final blend for tableting by blending the obtained granulates with extragranular components comprising fillers, disintegrants, lubricants or glidants;
   c. compressing the blend obtained from step b into tablet cores; and
   d. film coating the tablet cores obtained from step c.
56. The process of any one of Items 47 to 55, wherein the wet granulation is performed in a process of fluid bed granulation, low shear granulation or high shear granulation.

### Description of further advantages and preferred embodiments of the invention

The present invention is now described in more detail by preferred embodiments and examples, which are however presented for illustrative purpose only.

The object of the present invention was to provide an improved pharmaceutical composition with higher dissolution rate of drug substance apixaban, as well as a production process thereof. In one aspect the present invention provides a pharmaceutical composition comprising apixaban and a polymer having low viscosity. In another aspect the present invention provides a wet granulation process of manufacturing a pharmaceutical composition comprising apixaban and a polymer having low viscosity.

It was surprisingly found that a pharmaceutical composition comprising apixaban and a polymer having low viscosity, owing to its use as a wet granulation binder in a prior formulation process, results in an improved dissolution rate.

WO 2011/106478 describes that the size of apixaban particles is controlled to have a d_{0.9} equal to or less than 89 µm in order to achieve appropriate dissolution rate. However, for controlling particle size of drug substance, additional process such as milling or micronization of drug substance is necessary. This may not be advantageous from stability aspect because active drug substance can be degraded during such process. From financial aspect, this additional process may not also advantageous because micronization of drug substance is expensive. Unexpectedly, it was found that a pharmaceutical composition comprising polymer having low viscosity as a wet granulation binder showed improved dissolution rate of apixaban, without a need, let alone a limitation to control particle size of drug substance apixaban. Similarly, by using the polymer having low viscosity as a wet granulation binder, the desired pharmaceutical composition that exhibits appropriate dissolution rate is prepared according to the present invention. Therefore, the pharmaceutical composition comprising apixaban according to the present invention can be used with less limitations regarding particle size distribution of active substance.

The term "apixaban" used herein comprises 1-(4-methoxyphenyl)-7-oxo-6-[4-(2-oxopiperidin-1-yl)phenyl]-4,5,6,7-tetrahydro-1H pyrazolo[3,4-c]pyridin-3-carbamid in accordance with formula (1) above. Further, the term "apixaban" comprises all the pharmaceutically acceptable salts, hydrates and/or solvates thereof.

The term "low viscosity" used herein means a suitably low viscosity which enables the polymer having low viscosity used as a wet granulation binder in a wet granulation process to enhance the dissolution rate of pharmaceutical composition comprising apixaban. For instance, the dissolution rate of pharmaceutical composition may be enhanced by using the polymer having low viscosity as a wet granulation binder in a wet granulation process, if the pharmaceutical composition comprising apixaban and the polymer having low viscosity shows an improvement in the dissolution rate over a reference sample of (i) dry granulation which has otherwise similar, preferably the same ingredients in its composition, but is not prepared by wet granulation with polymer having low viscosity according to the present invention used as wet granulation binder (and its content may be replaced by an inert other excipient, or by a corresponding proportional increase in content of the other fixed ingredients). More preferably, such enhancement is demonstrated in comparison with a reference sample as defined in (i) but additionally containing a surfactant; more specifically, a reference sample defined by "Comparative example A or B" described below could be used for the preferred enhancement test of the low viscosity wet binder.

In a preferred embodiment of the present invention the low viscosity means a dynamic viscosity of 2% aqueous solution at 20°C up to about 25 mPa·s. In a more preferred embodiment of the present invention, for a given polymer type, a species having relatively lower viscosity is selected, measured as a dynamic viscosity of 2% aqueous solution at 20°C.

In the present invention, the viscosity parameter may be measured under conditions described in monographs in European Pharmacopoeia (PhEur) or United States Pharmacopoeia and National formulary (USP and NF). Proposed measurement methods and conditions for particular polymers are as follows:
1) Povidone: 10% aqueous solution at 25°C up to about 10 mPa·s, determined according to:
   a) PhEur (PhEur monograph: Povidone, 2.2.8. Viscosity, and 2.2.9. Capillary viscometer method) and
   b) USP (USP monograph: Povidone and General Chapters: <911> Viscosity-capillary viscometer methods);
2) Hypromellose: 2% aqueous solution at 20°C up to about 20 mPa·s, determined according to:
   a) PhEur (PhEur monograph: Hypromellose, 2.2.8. Viscosity, and 2.2.9. Capillary viscometer method) and
   b) USP (USP monograph: Hypromellose and General Chapters: <911> Viscosity-capillary viscometer methods);
3) Hydroxypropyl cellulose:
   a) 2% aqueous solution at 20°C up to about 25 mPa·s, determined according to:
      i) PhEur (PhEur monograph: Hydroxypropyl cellulose, 2.2.10. Viscosity - rotating viscometer method) and
      ii) USP/NF (NF monograph: Hydroxypropyl cellulose and General Chapters: <912> Rotational rheometer methods);
   b) 5% aqueous solution at 25°C up to about 150 mPa·s, determined according to USP/NF (NF monograph: Hydroxypropyl cellulose and General Chapters: <912> Rotational rheometer methods);
   c) 10% aqueous solution at 25°C up to about 700 mPa·s, determined according to USP/NF (NF monograph: Hydroxypropyl cellulose and General Chapters: <912> Rotational rheometer methods);
4) Polyvinyl alcohol: 4% aqueous solution at 20°C up to about 40 mPa·s, determined according to:
   a) PhEur (PhEur monograph: Poly(vinyl alcohol) and 2.2.49. Falling ball viscometer method) and
   b) USP (USP monograph: Polyvinyl alcohol and General Chapters: <913> Rolling ball viscometer methods);
5) Copovidone: 10% aqueous solution at 25°C up to about 10 mPa·s, determined according to:
   a) PhEur (PhEur monograph: Copovidone, 2.2.8. Viscosity, and 2.2.9. Capillary viscometer method) and
   b) USP (USP monograph: Povidone and General Chapters: <911> Viscosity-capillary viscometer methods); and
6) Polyvinyl alcohol-polyethylene glycol graft copolymer:
   a) 20% aqueous solution at 25°C up to about 250 mPa·s, determined according to:
      i) PhEur (PhEur monograph: Macrogol Poly(Vinyl Alcohol) Grafted Copolymer, 2.2.10. Viscosity - rotating viscometer method) and
      ii) USP/NF (NF monograph: Ethylene Glycol and Vinyl Alcohol Grafted Copolymer and General Chapters: <912> Rotational rheometer methods);
   b) 10% aqueous solution at 25°C up to about 20 mPa·s, determined according to USP/NF (General Chapters: <912> Rotational rheometer methods);
   c) 5% aqueous solution at 25°C up to about 10 mPa·s, determined according to USP/NF (General Chapters: <912> Rotational rheometer methods).

If desired, for standardization and validation of viscosity measurement for an unknown polymer, one may proceed as follows: take a commercially available sample polymer with product information in its specification or product brochure which specifies a known nominal viscosity value. By comparison with the nominal value, the viscosimeter conditions can be set to conform with the actually measured value. With the viscosimeter, so standardized and validated, viscosity of an unknown polymer sample can be carried out.

According to one embodiment of the present invention, the different types of povidone and copovidone may be characterized by their viscosity in aqueous solution, relative to that of water, expressed as a K-value. The K-value may be calculated from the concentration and viscosity of povidone or copovidone in aqueous solution, relative to that of water, for example, according to viscosity method and formula for calculation of K-value disclosed in PhEur (PhEur monographs: Povidone, Copovidone, 2.2.9. Capillary viscometer method) and USP (USP Monographs: Povidone, Copovidone, General Chapters: <911> Viscosity - capillary viscometer methods).

In a preferred embodiment of the present invention the polymer having low viscosity is selected from the group consisting of povidone, hypromellose, hydroxypropyl cellulose polyvinyl alcohol, copovidone, polyvinyl alcohol-polyethylene glycol graft copolymer, respectively having low viscosity, and a mixture thereof.

In another preferred embodiment of the present invention, the polymer having low viscosity has an average molecular weight of up to about 200000.

In a further aspect the polymer having low viscosity according to the present invention is povidone with a dynamic viscosity of 10% aqueous solution at 25°C up to about 10 mPa·s.

In a further aspect the polymer having low viscosity is povidone with K-value up to about 32. In another aspect the povidone with low viscosity has an average molecular weight of up to about 55000. In a more preferred embodiment the povidone having low viscosity is selected from the group consisting of K12, K17, K25, K30 and a mixture thereof. In the most preferred embodiment the povidone having low viscosity is K25.

In a further aspect the polymer having low viscosity is hypromellose with a dynamic viscosity of 2% aqueous solution at 20°C up to about 20 mPa·s. Preferably, the hypromellose with low viscosity has an average molecular weight of up to about 100000.

In a more preferred embodiment the hypromellose having low viscosity is selected from the group consisting of any of substitution types of hypromellose (2208, 2910, 2306), and a mixture thereof. Examples of hypromellose having low viscosity are Pharmacoat 603, Pharmacoat 645, Pharmacoat 606 and Pharmacoat 615, Pharmacoat 904, provided by Colorcon, as well as Methocel K3 Premium LV, Methocel E3 Premium LV, Methocel E5 Premium LV, Methocel E6 Premium LV and Methocel E15 Premium LV, provided by Shinetzu, well as Benecel E3, Benecel E6, Benecel E15, Benecel A15 LV provided by Ashland. In the most preferred embodiment the hypromellose having low viscosity is Pharmacoat 603.

In a further aspect the polymer having low viscosity is hydroxypropyl cellulose with a dynamic viscosity of 2% aqueous solution at 20°C up to about 25 mPa·s, a dynamic viscosity of 5% aqueous solution at 25°C up to about 150 mPa·s or a dynamic viscosity of 10% aqueous solution at 25°C up to about 700 mPa·s. Preferably, the hydroxypropyl cellulose with low viscosity has an average molecular weight of up to about 100000.

Examples of hydroxypropyl cellulose having low viscosity are Klucel ELF, Klucel EF, Klucel EXF and Klucel LF, provided by Aqualon, as well as Nisso HPC-LM, Nisso HPC-L, Nisso HPC-SL, Nisso HPC-SSL, provided by Nippon Soda Co.

In a further aspect the polymer having low viscosity is polyvinyl alcohol with a dynamic viscosity of 4% aqueous solution at 20°C up to about 40 mPa·s. Preferably, the polyvinyl alcohol with low viscosity has an average molecular weight of up to about 200000.

Examples of polyvinyl alcohol having low viscosity are PVA EMPROVE® 4-88, PVA EMPROVE® 5-88, PVA EMPROVE® 8-88, PVA EMPROVE® 28-99, PVA EMPROVE® 40-88, provided by Merck as well as Mowiol® 3-96, Mowiol® 4-88, Mowiol® 4-98, Mowiol® 9-98, Mowiol® 18-88, Mowiol® 40-88 provided by Sigma-Aldrich.

In a further aspect the polymer having low viscosity is copovidone (polyvinylpyrrolidone-vinyl acetate copolymer) with a dynamic viscosity of 10% aqueous solution at 25°C up to about 10 mPa·s.

In a further aspect the polymer having low viscosity is copovidone with K-value up to about 35. In another aspect the copovidone with low viscosity has an average molecular weight of up to about 80000. Examples of copovidone having low viscosity are Kollidon VA 64 and Luviskol VA, provided by BASF, as well as Plasdone S-630, provided by ISP.

In a further aspect the polymer having low viscosity is polyvinyl alcohol-polyethylene glycol graft copolymer (Ethylene Glycol and Vinyl Alcohol Grafted Copolymer, Macrogol Poly(Vinyl Alcohol) Grafted Copolymer) with a dynamic viscosity of 20% aqueous solution at 25°C up to about 250 mPa·s, a dynamic viscosity of 10% aqueous solution at 25°C up to about 20 mPa·s or a dynamic viscosity of 5% aqueous solution at 25°C up to about 10 mPa·s.

Preferably, the polyvinyl alcohol-polyethylene glycol graft copolymer with low viscosity has an average molecular weight of up to about 60000. Examples of polyvinyl alcohol-polyethylene glycol graft copolymer having low viscosity include Kollicoat IR, provided by BASF.

In a further aspect a combination of any of above-listed polymers having low viscosity may be employed in the present invention.

The term "binder" used herein means any ingredients which ensure that tablets and/or granulates can be formed with required properties in the context of conventional pharmaceuticals, i.e. typically hold ingredients together, preferably with a desired mechanical strength, and give volume to the composition. In a preferred embodiment the pharmaceutical compositions according to the present invention comprise the polymer having low viscosity as binder, preferably as a wet granulation binder. In a further aspect the pharmaceutical compositions according to the present invention may comprise any further conventional binders used in pharmaceutical industry.

In a further aspect the present invention provides a pharmaceutical composition comprising apixaban, a polymer having low viscosity as binder and a surfactant.

Any surfactants used in pharmaceutical industry can be used in the present invention. In a preferred embodiment the surfactant in the pharmaceutical composition according to the present invention is selected from the group consisting of polyoxyethylene stearates, polyoxyethylene alkyl ethers, sorbitan fatty acid esters, poloxamers, polyoxyethylene castor oil derivatives, phospholipids, sodium lauryl sulphate, polysorbate (polyoxyethylene sorbitan fatty acid esters) and a mixture thereof, preferably sodium lauryl sulphate and polysorbate.

In another aspect of the present invention provides a pharmaceutical composition comprising apixaban having a particle size distribution of d_{0.9} 5 - 150 µm, preferably 30 - 130 µm, more preferably 40 - 120 µm. In a more preferred embodiment the pharmaceutical composition according to the subject invention comprises apixaban having a particle size distribution of d_{0.9} 50 - 100 µm.

The pharmaceutical compositions described herein can further contain tableting fillers such as lactose, microcrystalline cellulose, starch, starch derivatives, sugars, sugar alcohols, inorganic fillers such as calcium hydrogen phosphate or a mixture thereof.

The compositions described herein may also comprise disintegrants, such as crosscarmellose sodium, sodium starch glycolate, other starch derivatives, crospovidone, low substituted hydroxypropyl cellulose or a mixture thereof, as well as disintegration aids such as ion exchange resin such as Polacrilin Potassium and Magnesium Aluminometasilicate.

The compositions described herein may also comprise glidants, such as talc, silicon dioxide, magnesium silicate, sodium stearate or a mixture thereof.

The compositions described herein may also comprise lubricants, such as magnesium stearate, sodium stearyl fumarate, glyceryl behenate, stearic acid, calcium stearate or a mixture thereof.

The compositions described herein may also comprise film coating agents, such as polyvinyl alcohol, hypromellose, hydroxypropyl cellulose or other polymers used in pharmaceutical industry for film coating, or a mixture thereof.

In a further aspect the present invention provides a pharmaceutical composition comprising apixaban exhibiting dissolution properties that more than 80%, preferably more than 85%, more preferably more than 90% of apixaban is dissolved in 30 minutes in 900 ml of 0.1 M HCl solution. In the present invention, the dissolution testing may be performed according to any conventional methods, for example in an aqueous media buffered to an appropriate pH range without addition of a surfactant at an appropriate temperature (about 37±1 °C). In a preferred embodiment of the present invention the dissolution testing is performed in 900 mL of 0.1 M HCl solution at 37°C, using USP Apparatus 2 (paddles) method at a rotation speed of 75 rpm.

In another aspect, the present invention further provides a pharmaceutical composition for use in treating or preventing a thromboembolic disorder.

In another aspect, the present invention further provides a process for preparing the pharmaceutical composition comprising apixaban and a polymer having low viscosity.

In a preferred embodiment the pharmaceutical composition according to the subject invention is prepared by a wet granulation process, followed by preparation of final tableting blend, compaction step and coating step.

Surprisingly, it has been found that when using a wet granulation process wherein a polymer having low viscosity is employed as a wet granulation binder, the pharmaceutical composition comprising apixaban showed an improved dissolution rate. Prior art teaches apixaban formulations made using a wet granulation process resulted in lower dissolution compared to that made using a dry granulation process. However, it was surprisingly found that the use of a polymer having low viscosity as a wet granulation binder enables the pharmaceutical composition comprising apixaban to exhibit higher dissolution rate than the composition prepared by dry granulation process that was preferred according to the prior art. Therefore, very advantageously, by using the polymer having low viscosity in a wet granulation process, appropriate dissolution rate can be achieved with higher particle size of apixaban than without using the polymer having low viscosity in a wet granulation process. Prior art requires the particle size of apixaban to be controlled, which is disadvantageous from stability as well as financial aspects. Accordingly, it has been found to be advantageous when the apixaban composition comprises a polymer having low viscosity in view of its contribution to the dissolution rate of drug substance.

In a preferred embodiment the process for preparing the pharmaceutical composition comprises:
applying water or a granulation dispersion onto a blend comprising apixaban and/or a polymer having low viscosity,
wherein the granulation dispersion is prepared by dispersing apixaban and/or a polymer having low viscosity in a solvent.

In a further aspect the process for preparing the pharmaceutical composition comprises further adding pharmaceutically acceptable excipients or a surfactant or both, to a granulation dispersion or to a blend comprising apixaban and/or a polymer having low viscosity.

In a more preferred embodiment the process for preparing the pharmaceutical composition comprises:
a. providing a granulation dispersion wherein apixaban and/or a polymer having low viscosity is dissolved or dispersed in water; and
b. providing wet granulates by spraying or sprinkling said granulation dispersion obtained from step a onto a blend comprising apixaban and/or a polymer having low viscosity, and pharmaceutically acceptable excipients.

In a more preferred embodiment the process for preparing the pharmaceutical composition comprises providing wet granulates by spraying or sprinkling water onto a blend comprising apixaban, a polymer having low viscosity and pharmaceutically acceptable excipients.

In a more preferred embodiment the process for preparing the pharmaceutical composition comprises:
a. providing a granulation dispersion wherein apixaban and/or a polymer having low viscosity and/or a surfactant is dissolved or dispersed in water; and
b. providing wet granulates by spraying or sprinkling said granulation dispersion obtained from step a onto a blend comprising apixaban and/or a polymer having low viscosity and/or a surfactant, and pharmaceutically acceptable excipients.

In a more preferred embodiment the process for preparing the pharmaceutical composition comprises providing wet granulates by spraying or sprinkling water onto a blend comprising apixaban, a polymer having low viscosity, a surfactant and pharmaceutically acceptable excipients.

In a further aspect the process of the present invention can further comprise the steps of:
a. drying wet granulates obtained after spraying or sprinkling;
b. providing a final blend for tableting by blending the obtained granulates with extragranular components comprising fillers, disintegrants, lubricants or glidants;
c. compressing the blend obtained from step b into tablet cores; and
d. film coating the tablet cores obtained from step c.

Preferably, the wet granulation process of the subject invention is performed in a process of fluid bed granulation, low shear granulation or high shear granulation.

In a further aspect, final blend for tableting is prepared by blending of obtained granules with optional extragranular components, such as fillers, disintegrants or glidants, and is then lubricated. The blending with extragranular excipients can be performed manually or by convection or diffusion mixer (double cone blender or cubic blender). Lubricated blend is then compressed into tablet cores. Compression step of the final blend into tablets can be performed by rotary and eccentric tableting machine. Tablet cores obtained this way can be coated with film coating that can be performed in perforated or conventional coating drums, with the film coating based on polyvinyl alcohol, hypromellose, hydroxypropyl cellulose or other polymers used in pharmaceutical industry for film coating.

### Examples

Examples 1 to 4 and Comparative example A were prepared with drug substance with higher particle size distribution (d_{0.9} 82 µm).

### Example 1

| Component | Amount per tablet (mg) | Portion |
|---|---|---|
| Apixaban (API) | 2.500 | 2.50% |
| Povidone K 25 (PVP) | 4.000 | 4.00% |
| Sodium lauryl sulphate (SDS) | 2.500 | 2.50% |
| Demineralized water* | 72.000 | - |
| Lactose monohydrate | 50.000 | 50.00% |
| Microcrystalline cellulose | 36.250 | 36.25% |
| Crosscarmellose sodium | 2.000 | 2.00% |
| Total granulate (dried) | 97.250 | 97.25% |
| Crosscarmellose sodium | 2.000 | 2.00% |
| Megnesium stearate | 0.750 | 0.75% |
| Total tablet core | 100.000 | 100.00% |

| | | |
|---|---|---|
| *removed during drying phase | | |

Sodium lauryl sulphate and povidone were dissolved in water. Apixaban was dispersed in obtained solution. This dispersion was sprinkled onto blend of lactose monohydrate, microcrystalline cellulose and crosscarmellose sodium in mortar and granulated with pestle so that wet granules were obtained. Obtained granules were dried in vacuum dryer and passed through screen.

Final blend for tableting was prepared by blending the obtained granules with disintegrant and lubricant manually. Final blend was then compressed into tablet cores by eccentric tableting machine.

### Example 2

| Component | Amount per tablet (mg) | Portion |
|---|---|---|
| Apixaban (API) | 2.500 | 2.50% |
| Hypromellose Pharmacoat 603 (HPMC) | 4.000 | 4.00% |
| Polysorbate 80V | 2.500 | 2.50% |
| Demineralized water* | 72.000 | - |
| Lactose monohydrate | 50.000 | 50.00% |
| Microcrystalline cellulose | 36.250 | 36.25% |
| Crosscarmellose sodium | 2.000 | 2.00% |
| Total granulate (dried) | 97.250 | 97.25% |
| Crosscarmellose sodium | 2.000 | 2.00% |
| Megnesium stearate | 0.750 | 0.75% |
| Total tablet core | 100.000 | 100.00% |

| | | |
|---|---|---|
| *removed during drying phase | | |

Polysorbate and hypromellose were dissolved in water. Apixaban was dispersed in obtained solution. This dispersion was sprinkled onto blend of lactose monohydrate, microcrystalline cellulose and crosscarmellose sodium in mortar and granulated with pestle so that wet granules were obtained. Obtained granules were dried in vacuum dryer and passed through screen.

Final blend for tableting was prepared by blending obtained granules with disintegrant and lubricant manually. Final blend was then compressed into tablet cores by eccentric tableting machine.

### Example 3

| Component | Amount per tablet (mg) | Portion |
|---|---|---|
| Apixaban (API) | 2.500 | 2.50% |
| Povidone K 25 (PVP) | 4.000 | 4.00% |
| Sodium lauryl sulphate (SDS) | 1.000 | 1.00% |
| Demineralized water* | 80.000 | - |
| Lactose monohydrate | 50.000 | 50.00% |
| Microcrystalline cellulose | 37.750 | 37.75% |
| Crosscarmellose sodium | 2.000 | 2.00% |
| Total granulate (dried) | 97.250 | 97.25% |
| Crosscarmellose sodium | 2.000 | 2.00% |
| Megnesium stearate | 0.750 | 0.75% |
| Total tablet core | 100.000 | 100.00% |

| | | |
|---|---|---|
| *removed during drying phase | | |

Sodium lauryl sulphate and povidone were dissolved in water. Apixaban was dispersed in obtained solution. This dispersion was sprayed onto blend of lactose monohydrate, microcrystalline cellulose and crosscarmellose sodium in top spray fluid bed equipment so that wet granules were obtained. Obtained granules were dried in fluid bed equipment and passed through screen.

Final blend for tableting was prepared by blending obtained granules with disintegrant and lubricant manually. Final blend was then compressed into tablet cores by eccentric tableting machine.

### Example 4

| Component | Amount per tablet (mg) | Portion |
|---|---|---|
| Apixaban (API) | 2.500 | 2.50% |
| Povidone K 25 | 4.000 | 4.00% |
| Lactose monohydrate | 50.000 | 50.00% |
| Microcrystalline cellulose | 38.750 | 38.75% |
| Crosscarmellose sodium | 2.000 | 2.00% |
| Demineralized water* | 36.000 | - |
| Total granulate (dried) | 97.250 | 97.25% |
| Crosscarmellose sodium | 2.000 | 2.00% |
| Megnesium stearate | 0.750 | 0.75% |
| Total tablet core | 100.000 | 100.00% |

| | | |
|---|---|---|
| *removed during drying phase | | |

Water was sprinkled onto blend of Apixaban, povidone, lactose monohydrate, microcrystalline cellulose and crosscarmellose sodium in mortar and granulated with pestle so that wet granules were obtained. Obtained granules were dried in vacuum dryer and passed through screen.

Final blend for tableting was prepared by blending of obtained granules with disintegrant and lubricant manually. Final blend was then compressed into tablet cores by eccentric tableting machine.

### Comparative example A

Comparative example A was prepared based on composition and process disclosed in WO 2011/106478 (Table 3). Dry granulation process was performed by briquetting technological procedure, also known as double compression technology.

| Component | Amount per tablet (mg) | Portion |
|---|---|---|
| Apixaban | 2.500 | 2.50% |
| Lactose anhydrous | 49.250 | 49.25% |
| Microcrystalline cellulose | 42.000 | 42.00% |
| Crosscarmellose sodium | 2.000 | 2.00% |
| Sodium lauryl sulphate | 1.000 | 1.00% |
| Megnesium stearate | 0.500 | 0.50% |
| Crosscarmellose sodium | 2.000 | 2.00% |
| Megnesium stearate | 0.750 | 0.75% |
| Total tablet core | 100.000 | 100.00% |

Apixaban, lactose anhydrous, microcrystalline cellulose, crosscarmellose sodium and sodium lauryl sulphate were manually blended and passed through screen. This blend was lubricated with magnesium stearate and compressed into briquettes by eccentric tableting machine. Briquettes were milled by oscillating bar mill so that dry granules were obtained. Dry granules were lubricated with magnesium stearate and compressed into tablet cores by eccentric tableting machine.

### Dissolution tests for Examples 1 to 4 and Comparative example A

A dissolution test was performed to show improved dissolution rate of Examples 1, 2, 3 and 4 prepared by wet granulation process, in comparison to Comparative example A prepared by dry granulation process, known from prior art.

The dissolution test was performed in 900 ml of 0.1 M HCl solution at 37°C, using USP Apparatus 2 (paddles) method at a rotation speed of 75 rpm.

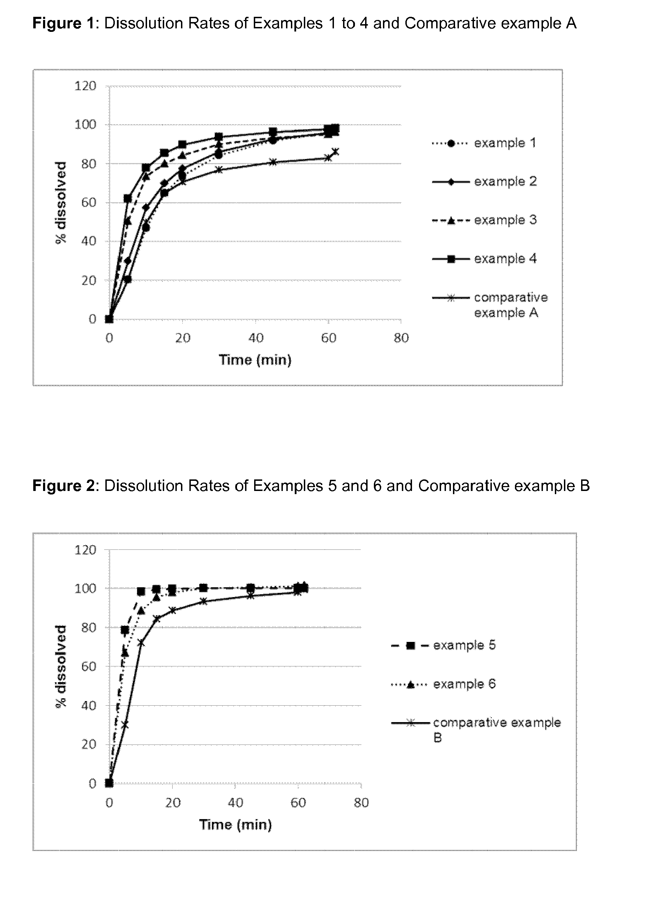

As illustrated in Figure 1, all four Examples 1, 2, 3 and 4 according to the subject invention showed improved dissolution rate compared to Comparative example A known from prior art. Examples 1, 2, 3 and 4 exhibited improved dissolution properties in that more than 84.4% apixaban was dissolved in 30 minutes in Examples 1 to 4, whereas only about 77% apixaban was dissolved in 30 minutes in Comparative example A.

Further, as can be seen from the dissolution results of Example 4, the subject invention showed high dissolution rate even without having a surfactant, owing to the use of a polymer having low viscosity as a wet granulation binder.

Meanwhile, Examples 5 and 6 and Comparative example B were prepared with drug substance with lower particle size distribution (d_{0.9} 17 µm).

### Example 5

| Component | Amount per tablet (mg) | Portion |
|---|---|---|
| Apixaban (API) | 2.500 | 2.50% |
| Povidone K 25 (PVP) | 4.000 | 4.00% |
| Lactose monohydrate | 50.000 | 50.00% |
| Microcrystalline cellulose | 37.750 | 36.25% |
| Crosscarmellose sodium | 2.000 | 2.00% |
| Sodium lauryl sulphate (SDS) | 1.000 | 2.50% |
| Demineralized water | 36.000 | - |
| Total granulate (dried) | 97.250 | 97.25% |
| Crosscarmellose sodium | 2.000 | 2.00% |
| Megnesium stearate | 0.750 | 0.75% |
| Total tablet core | 100.000 | 100.00% |

Sodium lauryl sulphate was dissolved in water. This solution was sprinkled onto blend of drug substance Apixaban, povidone, lactose monohydrate, microcrystalline cellulose and crosscarmellose sodium in mortar and granulated with pestle so that wet granules were obtained. Obtained granules were dried in vacuum dryer and passed through screen.

Final blend for tableting was prepared by blending of obtained granules with disintegrant and lubricant manually. Final blend was then compressed into tablet cores by eccentric tableting machine.

### Example 6

| Component | Amount per tablet (mg) | Portion |
|---|---|---|
| Apixaban (API) | 2.500 | 2.50% |
| Hypromellose Pharmacoat 603 (HPMC) | 4.000 | 4.00% |
| Sodium lauryl sulphate (SDS) | 1.000 | 1.00% |
| Demineralized water | 80.000 | - |
| Lactose monohydrate | 50.000 | 50.00% |
| Microcrystalline cellulose | 37.750 | 37.75% |
| Crosscarmellose sodium | 2.000 | 2.00% |
| Total granulate (dried) | 97.250 | 97.25% |
| Crosscarmellose sodium | 2.000 | 2.00% |
| Megnesium stearate | 0.750 | 0.75% |
| Total tablet core | 100.000 | 100.00% |

Sodium lauryl sulphate and HPMC were dissolved in water. Apixaban was dispersed in obtained solution. This dispersion was sprayed onto blend of lactose monohydrate, microcrystalline cellulose and crosscarmellose sodium in top spray fluid bed equipment so that wet granules were obtained. Obtained granules were dried in fluid bed equipment and passed through screen.

Final blend for tableting was prepared by blending of obtained granules with disintegrant and lubricant manually. Final blend was then compressed into tablet cores by eccentric tableting machine.

### Comparative example B

Comparative example B was prepared based on composition and process disclosed in WO 2011/106478 (Table 3). Dry granulation process was performed by briquetting technological procedure, also known as double compression technology.

| Component | Amount per tablet (mg) | Portion |
|---|---|---|
| Apixaban | 2.500 | 2.50% |
| Lactose anhydrous | 49.250 | 49.25% |
| Microcrystalline cellulose | 42.000 | 42.00% |
| Crosscarmellose sodium | 2.000 | 2.00% |
| Sodium lauryl sulphate | 1.000 | 1.00% |
| Megnesium stearate | 0.500 | 0.50% |
| Crosscarmellose sodium | 2.000 | 2.00% |
| Megnesium stearate | 0.750 | 0.75% |
| Total tablet core | 100.000 | 100.00% |

Apixaban, lactose anhydrous, microcrystalline cellulose, crosscarmellose sodium and sodium lauryl sulphate were manually blended and passed through screen. This blend was lubricated with magnesium stearate and compressed into briquettes by eccentric tableting machine. Briquettes were milled by oscillating bar mill so that dry granulates were obtained. Dry granules were lubricated with magnesium stearate and compressed into tablet cores by eccentric tableting machine.

### Dissolution tests for Examples 5 and 6 and Comparative example B

A dissolution test was performed to show improved dissolution rate of Examples 5 and 6 prepared by wet granulation process, in comparison to Comparative example B prepared by dry granulation process, known from prior art.

The dissolution test was performed in 900 ml of 0.1 M HCl solution at 37°C, using USP Apparatus 2 (paddles) method at a rotation speed of 75 rpm.

As illustrated in Figure 2, both Examples 5 and 6 according to the subject invention showed improved dissolution rate compared to Comparative example B according to prior art. Examples 5 and 6 exhibited improved dissolution properties in that more than 98% apixaban was dissolved in 30 minutes in Examples 5 and 6, which was much higher than the dissolution rate of Comparative example B.

Therefore, with both low particle size distribution (d_{0.9} 17 µm) and high particle size distribution (d_{0.9} 82 µm), pharmaceutical compositions according to the present invention were proven to achieve faster dissolution of drug substance compared to pharmaceutical compositions prepared according to prior art.

### Dissolution tests for Example 3 and Comparative example B

Another dissolution test was performed to compare the dissolution rate of Example 3 (pharmaceutical composition according to the present invention prepared with drug substance with higher particle size distribution of d_{0.9} 82 µm) with that of Comparative example B (pharmaceutical composition according to prior art prepared with drug substance with lower particle size distribution of d_{0.9} 17 µm).

The dissolution test was performed in 900 ml of 0.1 M HCl solution at 37°C, using USP Apparatus 2 (paddles) method at a rotation speed of 75 rpm.

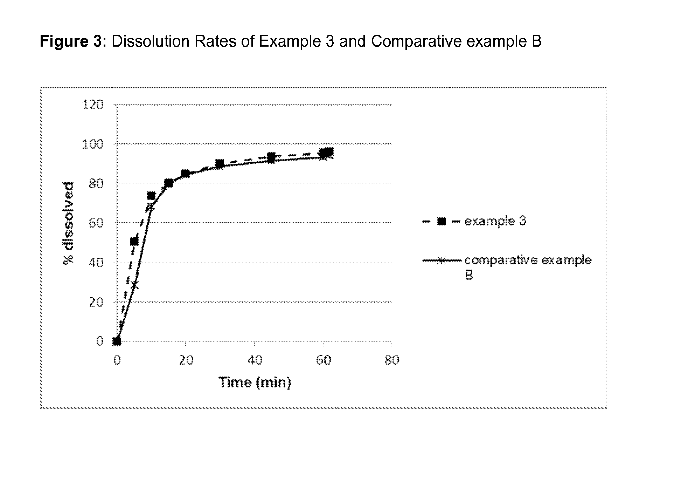

The results of dissolution test are illustrated in Figure 3. Surprisingly, the dissolution rate of pharmaceutical composition according to the present invention was comparable to the dissolution rate of pharmaceutical composition prepared according to prior art, even though the particle size distribution of the subject composition was much higher than that of the comparative composition (d_{0.9} 82 µm of Example 3 is almost 5 times higher than d_{0.9} 17 µm of Comparative example B).

Therefore, the pharmaceutical compositions according to the present invention provide superior dissolution rate compared to solutions disclosed in prior art. Such surprisingly new effects of the present invention enable that drug substance with higher particles can be also used for achieving appropriate dissolution rate of apixaban. More surprisingly but very advantageously, the pharmaceutical compositions according to the present invention provide high dissolution rate even without having a surfactant, by using a polymer having low viscosity as binder.

## Claims

1. A pharmaceutical composition comprising apixaban and a polymer having low viscosity as binder.

2. The pharmaceutical composition of claim 1, wherein the polymer having low viscosity has a dynamic viscosity of 2% aqueous solution at 20°C up to about 25 mPa·s.

3. The pharmaceutical composition of claim 1 or 2, wherein the polymer having low viscosity is selected from the group consisting of povidone, hypromellose, hydroxypropyl cellulose, polyvinyl alcohol, copovidone, polyvinyl alcohol-polyethylene glycol graft copolymer, respectively having low viscosity, and a mixture thereof.

4. The pharmaceutical composition of any one of preceding claims, wherein the polymer having low viscosity has an average molecular weight of up to about 200000.

5. The pharmaceutical composition of any one of preceding claims, wherein the polymer having low viscosity is povidone with a dynamic viscosity of 10% aqueous solution at 25°C up to about 10 mPa·s, or povidone with K-value up to about 32, or povidone having an average molecular weight of up to about 55000; preferably povidone is selected from the group consisting of K12, K17, K25, K30 and a mixture thereof, more preferably K25.

6. The pharmaceutical composition of any one of claims 1 to 4, wherein the polymer having low viscosity is hypromellose with a dynamic viscosity of 2% aqueous solution at 20°C up to about 20 mPa·s, or hypromellose having an average molecular weight of up to about 100000; preferably hypromellose is selected from the group consisting of substitution types of hypromellose 2208, 2910, 2306, and a mixture thereof, more preferably Pharmacoat 603.

7. The pharmaceutical composition of any one of claims 1 to 4, wherein the polymer having low viscosity is hydroxypropyl cellulose with a dynamic viscosity of 2% aqueous solution at 20°C up to about 25 mPa·s, or hydroxypropyl cellulose having an average molecular weight of up to about 100000.

8. The pharmaceutical composition of any one of claims 1 to 4, wherein the polymer having low viscosity is polyvinyl alcohol with a dynamic viscosity of 4% aqueous solution at 20°C up to about 40 mPa·s, or polyvinyl alcohol having an average molecular weight of up to about 200000.

9. The pharmaceutical composition of any one of claims 1 to 4, wherein the polymer having low viscosity is copovidone (polyvinylpyrrolidone-vinyl acetate copolymer) with a dynamic viscosity of 10% aqueous solution at 25°C up to about 10 mPa·s, or copovidone with K-value up to about 35, or copovidone having an average molecular weight of up to about 80000.

10. The pharmaceutical composition of any one of claims 1 to 4, wherein the polymer having low viscosity is polyvinyl alcohol-polyethylene glycol graft copolymer (Ethylene Glycol and Vinyl Alcohol Grafted Copolymer, Macrogol Poly(Vinyl Alcohol) Grafted Copolymer) with a dynamic viscosity of 20% aqueous solution at 25°C up to about 250 mPa·s, polyvinyl alcohol-polyethylene glycol graft copolymer with a dynamic viscosity of 10% aqueous solution at 25°C up to about 20 mPa·s, or polyvinyl alcohol-polyethylene glycol graft copolymer with a dynamic viscosity of 5% aqueous solution at 25°C up to about 10 mPa·s, or polyvinyl alcohol-polyethylene glycol graft copolymer having an average molecular weight of up to about 60000.

11. The pharmaceutical composition of any one of preceding claims, further comprising a surfactant.

12. A pharmaceutical composition as defined in any one of preceding claims for use in treating or preventing a thromboembolic disorder.

13. A process of manufacturing a pharmaceutical composition of any one of preceding claims, comprising mixing apixaban and a polymer having low viscosity; preferably involving a wet granulation process using said polymer having low viscosity as a wet granulation binder.

14. The process of claim 13, comprising:
applying water or a granulation dispersion onto a blend comprising apixaban and/or a polymer having low viscosity,
wherein the granulation dispersion is prepared by dispersing apixaban and/or a polymer having low viscosity in a solvent.

15. The process of any one of claims 13 to 14, comprising further adding pharmaceutically acceptable excipients or a surfactant or both, to a granulation dispersion or to a blend comprising apixaban and/or a polymer having low viscosity.
